# EUROPÄISCHE PATENTSCHRIFT

(11) **EP 0 218 885 B1**
(45) Veröffentlichungstag und Bekanntmachung des Hinweises auf die Patenterteilung: **24.02.1993**
(21) Anmeldenummer: 86112207.5
(22) Anmeldetag: 03.09.1986
(51) Int. Cl.: A61M 29/00

(54) **Dilatationskatheter**
Dilatation catheter
Cathéter de dilatation

(30) Priorität: 13.09.1985 DE 3532653
(43) Veröffentlichungstag der Anmeldung: 22.04.1987
(73) Patentinhaber: Kaltenbach, Martin, Prof. Dr. med., D-63303 Dreieich (DE)
(72) Erfinder: Kaltenbach, Martin, Prof. Dr. med., D-63303 Dreieich (DE)
(74) Vertreter: Patent- und Rechtsanwaltssozietät, Schmitt, Maucher & Börjes

(56) Entgegenhaltungen:
- EP-A- 0 117 519
- US-A- 3 557 794
- US-A- 4 030 503

## Beschreibung

Die Erfindung betrifft einen Dilatationskatheter gemäß dem Oberbegriff des Patentanspruches 1.

Ein Dilatationskatheter mit einer Federspirale und einem in seinen Abmessungen veränderbaren Arbeitsende ist aus der US-A-4 030 503 bekannt. Die Federspirale ist dabei am Arbeitsende zu einem Schneidelement vergrößert, welches bei der Vorwärtsbewegung durch eine Gefäßverengung Ablagerungen abtrennen soll, was nach neuerer Sicht wegen der Gefahr, die von losgelösten Teilchen innerhalb des Gefäßsystemes ausgehen kann, unerwünscht erscheint. Außerdem kann die eingängige Federspirale bei der Verdrehung innerhalb einer Gefäßverengung derart verdrillt werden, daß sie in ihrem Durchmesser verkleinert wird und somit nicht zu einer Aufweitung des Gefäßes auf mechanischem Wege im Sinne einer Dilatation führen kann.

Aus der EP-A-0 117 519 ist ein Katheter bekannt, welcher an seinem Arbeitsende mehrere Schälmesser aufweist, wobei die Verbindung für den Drehantrieb dieser Schälmesser aus einem Spanndraht und einem schraubenlinienförmig darauf gewickelten Band sowie einer Umhüllung besteht. Die Drehfestigkeit des Katheters zwischen dem Antrieb und den Schälmessern wird also durch einen Verbund mehrerer Einzelelemente erreicht.

Der Erfindung liegt die Aufgabe zugrunde, einen Dilatationskatheter gemäß dem Oberbegriff des Patentanspruches 1 so auszubilden, daß er leicht auch in feine Gefäße einführbar und dafür entsprechend biegsam ist und seine Form den jeweiligen Gegebenheiten im zu behandelnden Gefäß angepaßt werden kann, ohne daß ein gasförmiges oder flüssiges Medium für die Erweiterung eines Ballons erforderlich ist und ohne daß für die Erweiterung des Gefäßes ein Abtragen von Ablagerungen durchgeführt werden muß, wobei der Druckkörper die notwendige Festigkeit zum Aufweiten des Gefäßes haben soll.

Diese Aufgabe wird erfindungsgemäß dadurch gelöst, daß die drehbare Federspirale und der als Federspirale ausgebildete Druckkörper doppelläufig sind.

Zweckmäßig ist es dabei, wenn die Federspirale an ihrem distalen Ende aufgeweitet ist und so den Druckkörper bildet.

Die Drehung oder Rotation kann von Hand oder motorisch erfolgen. Zweckmäßig ist es dabei, wenn zum Drehen des Druckkörpers ein Elektromotor am Katheterende vorgesehen ist.

Weitere zweckmäßige Ausgestaltungen der Erfindung bezüglich der Veränderung des Durchmessers des Druckkörpers sind Gegenstand der Ansprüche 4 bis 6.

Es sei noch erwähnt, daß die doppelläufige Federspirale aus Stahldraht bestehen kann.

Nachstehend ist ein Ausführungsbeispiel der Erfindung anhand der Zeichnung näher erläutert. Es zeigt:
- Fig. 1: einen Dilatationskatheter nach der Erfindung, und
- Fig. 2: den Dilatationskatheter gemäß Figur 1 mit veränderten Abmessungen zum Beseitigen einer Engstelle.

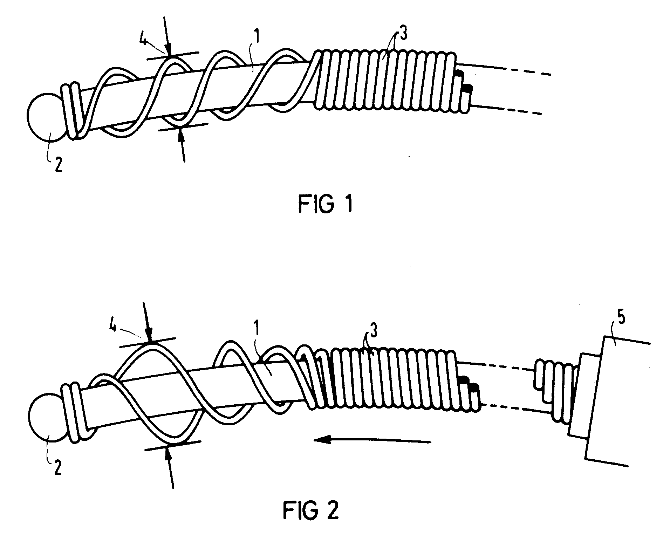

Der in den Figuren 1 und 2 dargestellte Dilatationskatheter weist einen flexiblen Draht 1 auf, der an seinem Ende mit einer Verdickung 2 versehen ist. Auf den Draht 1 ist eine Federspirale 3 aufgeschoben, die an ihrem distalen Ende aufgeweitet ist und so einen Druckkörper 4 bildet. Bei dem dargestellten Ausführungsbeispiel ist die Spirale 3 eine doppelläufige Stahldrahtspirale. Der Druckkörper 4 kann in seinem Durchmesser durch Stauchung erweitert werden. Die Stauchung wird durch Längszug erzeugt. Den Längszug bewirkt der Draht 1, der mit seiner Erweiterung 2 gegenüber der Spirale 3 nach rechts gezogen wird und so den Durchmesser des Druckkörpers 4 bei dem Beispiel von etwa 1,8 auf 4 mm vergrößert.

Für die Behandlung einer Engstelle wird der Katheter in der Pfeilrichtung in das verengte oder verschlossene Blutgefäß vorgeschoben, bis der Druckkörper 4 in der Engstelle liegt. Dabei wird die Spirale 3 durch einen am Katheterende vorgesehenen Elektromotor 5 in Rotation versetzt, so daß eine leichte Passage möglich ist. Zur Beseitigung der Engstelle wird bei fortdauernder Rotation der Spirale 3 und damit des Druckkörpers 4 Zug auf den Draht 1 ausgeübt und damit der Durchmesser des Druckkörpers 4 erweitert.

Die wesentlichen Vorteile des Beschriebenen Dilatationskatheters sind folgende:
1. Reibungsminderung in axialer Richtung durch Rotation.
2. Passagemöglichkeit auch engster Stenosen oder von Verschlüssen durch geringen Außendurchmesser und geringen Reibungsverlust infolge Wegfalls der Ballonhülle.
3. Variable Erweiterungsmöglichkeit durch Durchmesseränderung des Endstückes während des Eingriffes.
4. Keine Unterbrechung des Blutflusses während des Erweiterungsvorganges. Damit Vermeidung einer Mangeldurchblutung bei Organen, die für eine Unterbrechung der Blutzufuhr besonders empfindlich sind, wie Herz, Gehirn, Nieren, und dadurch besonders schonende, langsame Erweiterung von Engstellen ohne Zeitdruck.

Die Erweiterung des Druckkörpers 4 kann durch Verwendung elastischen Materials, Veränderung der Drehzahl (Fliehkraft) oder durch Zug auf den im Innern des Katheters laufenden Draht erfolgen. Es ist denkbar, daß durch entsprechende Drehzahlwahl auf einen Zug auf den Draht 1 vollkommen verzichtet werden kann.

## Patentansprüche

1. Dilatationskatheter mit einem am distalen Ende angebrachten, in seinen Abmessungen veränderbaren Druckkörper (4) zum Ausüben eines Druckes auf die eingeengte Gefäßwand, wobei der Druckkörper (4) aus elastischem Material besteht, drehbar gelagert und mit einem längs des Katheters verlaufenden, drehbaren, drehstabilen, als Federspirale (3) ausgebildeten Element verbunden ist, dadurch gekennzeichnet daß die drehbare Federspirale (3) und der als Federspirale ausgebildete Druckkörper (4) doppelläufig sind.

2. Dilatationskatheter nach Anspruch 1, dadurch gekennzeichnet, daß die Federspirale (3) an ihrem distalen Ende aufgeweitet ist und so den Druckkörper (4) bildet.

3. Dilatationskatheter nach Anspruch 1 oder 2, dadurch gekennzeichnet, daß zum Drehen des Druckkörpers (4) ein Elektromotor (5) am Katheterende vorgesehen ist.

4. Dilatationskatheter mit Mitteln zum Verändern des Durchmessers des Druckkörpers nach einem der Ansprüche 1 bis 3, dadurch gekennzeichnet, daß die Erweiterung des Durchmessers des Druckkörpers (4) über die Drehzahl durch Fliehkraft erfolgt.

5. Dilatationskatheter mit Mitteln zur Veränderung des Durchmessers des Druckkörpers nach einem der Ansprüche 1 bis 4, dadurch gekennzeichnet, daß der Druckkörper (4) einen Draht (1) umschließt und mit seinem distalen Ende an diesem Draht (1) derart anliegt, daß er durch einen Längszug auf den Draht (1) in seinem Durchmesser erweiterbar ist.

6. Dilatationskatheter nach Anspruch 5, dadurch gekennzeichnet, daß der flexible Draht (1) an seinem Ende eine Verdickung (2) aufweist.

7. Dilatationskatheter nach einem der Ansprüche 1 bis 6, dadurch gekennzeichnet, daß die doppelläufige Federspirale (3) aus Stahldraht besteht.

## Claims

1. A dilatation catheter having a pressure element (4) which is provided at the distal end, is changeable in its dimensions and serves to exert pressure on the constricted vascular wall, wherein the pressure element (4) consists of elastic material, is rotatably mounted and is connected to a rotatable, rotationally stable element which runs lengthwise of the catheter and takes the form of a spring spiral (3), **characterized in that** the rotatable spring spiral (3) and the pressure element (4) in the form of a spring spiral are double.

2. A dilatation catheter as claimed in claim 1, characterized in that the spring spiral (3) is expanded at its distal end and thus forms the pressure element (4).

3. A dilatation catheter as claimed in claim 1 or claim 2, characterized in that for rotating the pressure element (4) there is an electric motor (5) provided at the end of the catheter.

4. A dilatation catheter having means for changing the diameter of the pressure element, as claimed in any one of claims 1 to 3, characterized in that the enlargement of the diameter of the pressure element (4) takes place by way of the speed through centrifugal force.

5. A dilatation catheter having means for changing the diameter the pressure element, as claimed in any one of claims 1 to 4, characterized in that the pressure element (4) surrounds and with the distal end thereof rests against a wire (1) in such a way as to be enlargeable in its diameter by longitudinal travel of the wire (1).

6. A dilatation catheter as claimed in claim 5, characterized in that the flexible wire (1) has at the end thereof a thickening (2).

7. A dilatation catheter as claimed in any one of claims 1 to 6, characterized in that the double spring spiral (3) consists of steel wire.

## Revendications

1. Cathéter de dilatation comportant un corps-écarteur (4) disposé à l'extrémité distale et dont les dimensions sont variables, destiné à exercer une pression sur la paroi rétrécie d'un vaisseau, le corps-écarteur (4) étant constitué d'un matériau élastique, disposé de manière à tourner autour d'un axe et relié à un élément réalisé sous forme de spirale élastique (3), rotatif et stable, disposé le long du cathéter, caractérisé en ce que la spirale élastique (3) rotative et le corps-écarteur (4) réalisé sous forme de spirale élastique sont doubles.

2. Cathéter de dilatation selon la revendication 1, caractérisé en ce que la spirale élastique (3) est élargie à son extrémité distale pour former ainsi le corps-écarteur écarteur(4).

3. Cathéter de dilatation selon la revendication 1 ou 2, caractérisé en ce qu'un moteur électrique (5) est prévu à l'extrémité du cathéter pour faire tourner le (4).

4. Cathéter de dilatation avec possibilité de modifier le diamètre du corps-écarteur selon l'une des revendications 1 à 3, caractérisé en ce que la force centrifuge permet, par le biais de la vitesse de rotation, d'augmenter le diamètre du corps-écarteur (4).

5. Cathéter de dilatation avec possibilité de modifier le diamètre du corps-écarteur selon l'une des revendications 1 à 4, caractérisé en ce que le corps-écarteur (4) entoure une tige (1) et est en contact par son extrémité distale avec cette tige (1), de sorte que le diamètre de ce corps-écarteur peut être agrandi en exerçant une traction longitudinale sur la tige (1).

6. Cathéter de dilatation selon la revendication 5, caractérisé en ce que la tige (1) flexible présente à son extrémité un élargissement (2).

7. Cathéter de dilatation selon l'une des revendications 1 à 6, caractérisé en ce que la spirale élastique (3) double se compose d'un fil d'acier.
